# EUROPÄISCHE PATENTSCHRIFT

(11) **EP 2 022 503 B1**
(45) Veröffentlichungstag und Bekanntmachung des Hinweises auf die Patenterteilung: **19.10.2011**
(21) Anmeldenummer: 08161745.8
(22) Anmeldetag: 04.08.2008
(51) Int. Cl.: A61K 36/064, A61K 36/45, A23L 1/00, A61K 9/16

(54) **Verkapselte Vacciniumextrakte mit ausgewogener Magen-Darm-Freisetzung**
Encapsulated vaccine extracts with balanced intestinal release
Extraits de vaccin encapsulés ayant une libération équilibrée dans l'estomac-intestin

(30) Priorität: 07.08.2007 US 954370 P
(43) Veröffentlichungstag der Anmeldung: 11.02.2009
(73) Patentinhaber: Symrise AG, 37603 Holzminden (DE)
(72) Erfinder: Siegel, Sven, 37671 Höxter (DE); Krammer, Gerhard, 37603, Holzminden (DE); Mavric, Elvira, 37603, Holzminden (DE)
(74) Vertreter: Eisenführ, Speiser & Partner

(56) Entgegenhaltungen:
- WO-A-01/28526
- US-A1- 2008 255 226
- DATABASE WPI Week 200340 Thomson Scientific, London, GB; AN 2003-424039 XP002501903 & JP 2002 335881 A (YUKIJIRUSHI SHOKUHIN KK) 26. November 2002 (2002-11-26)

## Beschreibung

Anthocyane gehören zu den wichtigsten polyphenolischen Verbindungen in Beeren. Sie können bis zu 5000 mg/kg Frischgewicht in Beeren enthalten sein. Die in der Natur am häufigsten vorkommenden Aglycone (Anthocyanidine) sind: Pelargonidin, Cyanidin, Delphinidin, Peonidin, Petunidin, Malvidin. Die Anthocyanidine liegen in Pflanzen glycosidisch gebunden vor. Beeren enthalten ca. 15 verschiedene Anthocyane.

Anthocyane werden in besonders hoher Konzentration in den Früchten (Beeren) von Pflanzen der Gattung Vaccinium angetroffen. Einen besonders hohen Anthocyangehalt weisen die Früchte der Heidelbeere (*Vaccinium myrtillus*) auf. Auch die anderen Arten der Gattung Vaccinium weisen hohe Gehalte an Anthocyanen auf. Hierzu gehören die amerikanische Heidelbeere oder Kulturheidelbeere (*Vaccinium corymbosum*), Preiselbeere (*Vaccinium vitis-idaea*), gewöhnliche Moosbeere (*Vaccinium oxycoccos*), großfruchtige Moosbeere oder Kranbeere (cranberry) (*Vaccinium macrocarpon*), kleinfrüchtige Moosbeere (*Vaccinium microcarpum*), Alpen Rauschbeere (*Vaccinium gaultheroides*), Rauschbeere (*Vaccinium uliginosum*), Big Huckleberry (*Vaccinium membranaceum*), Red Huckleberry (*Vaccinium parvifolium),* Sparkleberry (*Vaccinium arboreum*), Ohelo-Beere (*Vaccinium reticulatum*), und die kanadische Blaubeere (*Vaccinium myrtilloides*).

Anthocyane können freie Radikale abfangen und durch das konjugierte Doppelbindungssystem stabilisieren, wodurch Radikal-Kettenreaktionen unterbrochen werden. Postuliert wurde eine Schutzfunktion den Proteinen gegenüber. Polyphenole verhindern bzw. hemmen die Bildung von Carbonylverbindungen, die in der Lage sind, mit freien Aminogruppen zu reagieren, und somit Proteine irreversibel zu verändern. Polyphenole reduzieren die Lipidperoxidation und fungieren als Radikalfänger (J. Agric. Food Chem., 2004, 52, 7419-7424: Inhibition of protein and lipid oxidation in liposomes by berry phenolics*,*). Dieser Effekt wurde *in vitro* eindeutig bestätigt, wobei sich die Anthocyane von Bilberry (Heidelbeere oder Blaubeere) am wirksamsten zeigten, gefolgt von Himbeeren, Lingobeeren und schwarzen Johannisbeeren. Die Aufnahme von frischen Erdbeeren (240 g), gefriergetrockneten Heidelbeeren (Bilberry) (100 g) und Beerensäften erhöht die antioxidative Kapazität des Blutplasmas bis zu 30 % (Current Nutr. & Food Sci., 2005, 1, 71-86: Potential Health Benefits of Berries).

Die Heidelbeere ist in der Naturmedizin für ihre heilende Wirkung bei Diarrhö und Magen-Darm-Erkrankungen bekannt, sie findet Anwendung in verschiedensten medizinischen Gebieten, z.B. Artherosklerose, Katarakt, Diabetes Mellitus, Diarrhoea oder Retinopatie.

Polyphenole bzw. Anthocyane werden auch im Zusammenhang mit Herzkrankheiten (CVD) und Krebs beschrieben. Sie sollen eine positive, vorbeugende Wirkung auf die Entwicklung dieser Krankheiten ausüben (Antioxidant Activity of Plant Extracts Containing Phenolic Compounds, J. Agric. Food. Chem., 1999, 47,3954-3962). In einer *in vitro* Studie wurden phenolische Extrakte verschiedener Beeren auf ihre Wirkung gegen die Zelllinien HT29 (Darm Krebszellen) und MCF7 (Brustkrebszellen) getestet. Es zeigte sich, dass alle Extrakte in Konzentrationen von 0,025 bis 0,5 % in zunehmender Reihenfolge hemmend auf das Zellwachstum wirkten (J. Agric. Food Chem., 2004, 52, 7264-7271: Inhibition of Cancer Cell Proliferation in Vitro by Fruit and Berry Extracts and Correlations with Antioxidant Levels). Des Weiteren konnte gezeigt werden, dass anthocyanreiche Lebensmittel *in vivo* und *in vitro* eine vorbeugende Wirkung auf verschiedene Krebsarten im GIT (Gastrointestinaltrakt) ausüben (oral, esophageal, intestinal, colorectal) (zusammengefasst in J. Agric. Food Chem, 2005, 53, 2859-2866: Analysis of Anthocyanins in Rat Intestinal Contents- Impact of Anthocyanin Chemical Structure on Fecal Excretion*).*

Aus der Volksmedizin ist es bekannt, dass Holunder- und Moosbeerenextrakte (Cranberries) zur Linderung von Harnwegserkrankungen eingesetzt werden. Diese Wirkung wird ebenfalls den Anthocyanen zugeschrieben.

### Bioverfügbarkeit

Für den Einsatz von Anthocyanen für medizinische Zwecke und zum Vorbeugen der Magen-Darm Krankheiten muss die Bioverfügbarkeit der Anthocyane gewährleistet sein. Diese hängt vor allem davon ab, wie und in welchem Umfang sie absorbiert und metabolisiert werden. Die Bioverfügbarkeit der Anthocyane liegt bei oraler Gabe bei Menschen im Durchschnitt bei 0,5 % (zusammengefasst in Fleschhut, J., Untersuchungen zum Metabolismus, zur Bioverfügbarkeit und zur antioxidativen Wirkung von Anthocyanen, Dissertation, 2004*).* Verglichen mit Flavonoiden weisen Anthocyane eine sehr geringe Bioverfügbarkeit auf. Für die Bioverfügbarkeit und die Kinetik der Anthocyane ist die β-D-glycosidische Form von entscheidender Bedeutung (J. Agric. Food Chem., 2006, 54, 583-589: Fate of Anthcyanins and Antioxidant Capacity in Contents of the Gastrointestinal Tract of Weanling Pigs Following Black Raspberry Consumption*;* J. Agric. Food Chem, 2005, 53, 2859-2866: Analysis of Anthocyanins in Rat Intestinal Contents- Impact of Anthocyanin Chemical Structure on Fecal Excretion,). Die beta-Glucosidasen, die im Körper vorkommen, spalten die glycosidischen Reste, und bilden Aglycone. Einige Studien zeigen, dass nach der oralen Verabreichung die glycosidische Form der Anthocyane absorbiert wird (Am. J. Clin. Nutr., 2001, 920-926: Anthocyanins are absorbed in glycated forms in elderly women: a pharmacokinetic study), aber auch in intakter unveränderter Form in Urin und Blut wieder gefunden wird.

Die Absorption einzelner Anthocyane ist durch die chemischen Eigenschaften des Aglycons und des Zuckerrestes bestimmt. Die Absorption der Anthocyane erfolgt sowohl im Magen als auch im Dünndarm (Current Nutr. & Food Sci., 2005, 1, 71-86: Potential Health Benefits of Berries).

WO 96/36433 beschreibt ein Verkapselungsprodukt mit einer Hülle, die von mikrobiellem Zellwandmaterial abgeleitet wird. Verkapselt wird eine Substanz, die nicht natürlich in der Mikrobe vorkommt. Zusätzlich ist die Hülle mit einem Farbstoff so gefärbt, dass die Färbung im Produkt sichtbar ist. Zur Herstellung des Produkts wird eine gewachsene und intakte Mikrobe mit der zu verkapselnden Substanz und einem Farbstoff in Kontakt gebracht. Die Mikrobe ist zu Beginn des Verkapselungsprozesses bevorzugt lebend und kann an ihrer Vermehrungsfähigkeit z.B. durch Bestrahlung gehindert werden. In jedem Fall muss die Mikrobe intakt sein und darf nicht lysiert vorliegen. Die während der Verkapselung in Lösung gegangene Zellsubstanz wird vor der Trocknung durch Zentrifugieren abgetrennt.

US 5,288,632 und EP 0 242 135 beschreiben die Herstellung eines mikrobiell verkapselten Materials in einer gewachsenen, intakten Mikrobe mit einem Fettgehalt kleiner als 40%, wobei die Einkapselung in Abwesenheit eines Plasmolysers bzw. einer organischen Fett-Erweiternden Substanz erfolgt. Die Mikrobe liegt in gewachsenen Form vor, d.h. sie wurde aus dem Kulturmedium gewonnen und ist intakt (nicht lysiert). Während der Herstellung diffundiert der zu verkapselnde Stoff durch die Zellwand hindurch und wird passiv innerhalb der Mikrobe zurückgehalten.

EP 0 453 316 beschreibt ein Verfahren zur Verkapselung lipophiler Substanzen innerhalb von Hefezellen, deren intrazelluläre Komponenten durch eine Temperaturbehandlung im Bereich zwischen 30 und 100°C für mindestens eine Stunde, in Anwesenheit oder in Abwesenheit eines Elutionspromotors eluiert wurden.

Hierbei wird bevorzugt der Hefeextrakt vor dem Kontakt mit der lipophilen Substanz abgetrennt.

EP 1 454 534 beschreibt Mikrokapseln aus Mikroorganismen, die Fremdstoffe in den Mikroorganismen eingekapselt haben und auf deren Oberfläche Saccharide, Süßstoffe, Proteine und Zuckeralkohole angelagert sind. In dem Verfahren werden zuerst die Fremdstoffe in die Mikroorganismen eingelagert, und dann die Saccharide, Süßstoffe, Proteine und Zuckeralkohole angelagert. Die angelagerten Substanzen bewirken eine Veränderung des Freisetzungsverhaltens.

EP 0 566 347 beschreibt die Verkapselung von Farbstoffen in Hefe-Ghosts. Hierzu wird Hefe-Debris in einem speziellen Verfahren gereinigt, um anschließend die Farbstoffe einzukapseln. Während des Verfahrens werden die bei der Autolyse ausgetretenen Zellsubstanzen entfernt.

WO 2006/83666 beschreibt Verkapselung einer Mischung bestehend aus 50 mg Curcumin, 60 mg Heidelbeerextrakt, 250 mg Traubenkernextrakt, 375 mg Grünteeextrakt und 125 mg Apfelextrakt in Gelatinekapseln. Die Anwendung ist als Nahrungsergänzungsmittel zur Vorbeugung und Linderung der kardiovasculären Krankheiten und Entzündungsvorgängen beschrieben.

WO 2006/43858 beschreibt Anwendung von Mischungen verschiedener Pflanzenextrakte, die z.B. rote Heidelbeere (2 - 4 %) beinhalten, zur Entgiftung von Zellen, Erhöhung des antioxidativen Status des Körpers und Stimulation der intrazellulären Biotransformation der Xenobiotika. Die Anwendung erfolgt in Form von Gelatinekapseln.

RU 2 257 909 beschreibt Anwendung von Heidelbeerextrakt mit einem Flavonoidgehalt von 5 Gew.-%. Die Verabreichung erfolgt auf einem pharmazeutischen Trägerstoff in Form von Kapseln, Tabletten, Granulaten u. ä. Das Produkt hat eine medizinische Anwendung.

EP 1 537 789 beschreibt Mikroverkapselung von Karotenen, Tocopherolen, Extrakten der *Passiflora incarnata* (Apigenin, Luteolin, oder die enthsprechenden Glycoside) und Extrakten der *Vaccinium myrtillus* (Delphinidin). Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

### Diskussion des Standes der Technik

Viele der im Stand der Technik beschriebenen Verkapselungsprodukte weisen eine Freisetzungscharakteristik auf, die entweder nur zur Freisetzung der Wirkstoffe im Magen oder nur im Darm führt. So führen sehr gut wasserlösliche Trägerstoffe zu einer meist sofortigen Freisetzung der Wirkstoffe im Mund bzw. Magen. Nach der Freisetzung sind die Wirkstoffe sofort Abbauvorgängen ausgesetzt oder werden zum Teil irreversibel an Proteine gebunden. Die Verfügbarkeit im Darm ist dann nicht mehr gegeben. Auf der anderen Seite sind Verkapselungen, die im Darm freigesetzt werden sollen so beschaffen, dass sie die Wirkstoffe nicht im Magen freisetzen und dort keine Wirkung erzielen. Zum Zwecke der Gesunderhaltung und Vorbeugung von Krankheiten wäre es aber vorteilhaft, wenn die anthocyanhaltigen Extrakte sowohl im Magen als auch im Darm kontinuierlich freigesetzt werden würden.

Die Verkapselung von anthocyanhaltigen Extrakten in Hefen ist nach dem Stand der Technik mit Problemen verbunden. Für die Verkapselung ist es wichtig, dass ein Teil des Zellinhalts durch das Wirken der zelleigenen Enzyme abgebaut und aufgelöst wird. Beim Kontakt von lebenden Hefen mit den anthocyanhaltigen Extrakten werden die für die Autolyse wichtigen Enzyme inaktiviert oder gehemmt und der Abbau der Zellsubstanz findet nicht in dem gewünschten Ausmaß statt. Die anthocyanhaltigen Extrakte verbleiben daher überwiegend auf der Oberfläche der Zellen und werden ungenügend eingekapselt. Der Stand der Technik offenbart des weiteren Verkapselungsverfahren und daraus hergestellte Produkte, in denen die Hefen vor dem Kontakt mit den zu verkapselnden Substanzen eluiert bzw. autolysiert werden, wobei der aus den Zellen ausgetretene Extrakt vor dem Kontakt mit den zu verkapselnden Substanzen entfernt wird. Die so gewonnenen Verkapselungsprodukte weisen zwar eine Freisetzung im Magen auf, die Freisetzung im Darm ist aber sehr beschränkt.

Nach dem Stand der Technik zur Hefeverkapselung werden die zu verkapselnden Stoffe im Inneren der Hefezelle zurückgehalten.

Es war deshalb die Aufgabe der Erfindung, eine Zubereitung (Verkapselungsprodukt) für Anthocyane anzugeben, das eine gleichmäßige oder steuerbare Freisetzung der Anthocyane in Magen und Darm eines Säugetiers, insbesondere eines Menschen, ermöglicht. Die Herstellung der Zubereitung sollte möglichst einfach und wirtschaftlich durchführbar sein.

### Zusammenfassung der Erfindung

Erfindungsgemäß wird deshalb insbesondere eine Vacciniumfruchtextrakt-Zubereitung angegeben, umfassend:
a) einen Vacciniumfruchtextrakt,
b) einen Anteil nicht-wasserlöslicher Bestandteile eines Hefezell-Lysats, und
c) einen Anteil wasserlöslicher Bestandteile eines Hefezell-Lysats.

Die Erfinder nehmen an, dass der Vacciniumfrüchteextrakt reversibel an den wasserlöslichen Bestandteilen des Hefezell-Lysats gebunden vorliegt, ferner wird vermutet, dass der Vacciniumfruchtextrakt und die wasserlöslichen Bestandteilen des Hefezell-Lysats an die Oberfläche der unlöslichen Zellreste angelagert sind.

Die Freisetzung des verkapselten Vacciniumfruchtextrakts, insbesondere des Heidelbeerextrakts, findet in geeigneten Ausführungsformen ausgewogen im Magen und im Darm statt.

Des Weiteren wird ein Verfahren zur Herstellung des erfindungsgemäßen Verkapselungsproduktes und dessen Anwendung in Lebensmitteln angegeben.

Das Herstellungsverfahren umfasst die Schritte:
a) Bereitstellen eines Vacciniumfruchtextrakts,
b) Bereitstellen eines Anteils nicht-wasserlöslicher Bestandteile und eines Anteil wasserlöslicher Bestandteile eines Hefezell-Lysats, und
c) Mischen des Vacciniumfruchtextrakts mit den Hefezell-Lysatanteilen und Wasser bei einer Temperatur zwischen 0 und 60°C für einen Zeitraum bis zu 4 Stunden, vorzugsweise zwischen 6 und 30 min.

Erfindungsgemäße Verkapselungsprodukte finden vorzugsweise Anwendung in Lebensmitteln und pharmazeutischen Produkten.

### Detaillierte Beschreibung der Erfindung.

VacciniumfruchtextraktVacciniumfruchtextraktÜberraschend wurde herausgefunden, dass die erfindungsgemäße Zubereitung (das erfindungsgemäße Verkapselungsprodukt) sowohl im Magen als auch im Darm Vacciniumfruchtextrakte sehr gut freisetzt. Darüber hinaus findet die Freisetzung im Magen und im Darm in einem ausgewogenen Verhältnis statt. Diese Freisetzungscharakteristik ist überraschend, da die Verkapselung von Vacciniumfruchtextrakten in unlöslichen Zellresten allein bzw. in löslichen Bestandteilen des Hefelysat allein jeweils nur zu einer starken Freisetzung im Magen führt. Die Freisetzung im Darm verläuft in beiden Fällen nur sehr verzögert. Erst durch die Kombination des unlöslichen Zellrests, dem löslichen Anteil des Hefelysats und den Vacciniumfruchtextraktn wird die gewünschte Freisetzungscharakteristik erzielt. Das erfindungsgemäße Verkapselungsprodukt zeichnet sich im Gegensatz zum allgemeinen Stand der Technik dadurch aus, dass die Vacciniumfruchtextrakte nicht im Inneren des Hefezellrests gebunden vorliegen, sondern reversibel an die löslichen Bestandteile des Hefezell-Lysats gebunden und an die Oberfläche der unlöslichen Zellreste angelagert ist.

Vorteilhaft neben der speziellen Freisetzungscharakteristik sind die einfache Gestaltung des Herstellverfahrens erfindungsgemäßer Zubereitungen (Verkapselungsprodukten) wegen der Abwesenheit eines gesondert aufzutragenden Coatings sowie die hohe Umweltfreundlichkeit des Verfahrens, da bei der Verkapselung keine Abfälle entstehen. Zusätzlich ist der Verkapselungsschritt innerhalb kürzester Zeit durchführbar, sodass die wertvollen Inhaltsstoffe der Vacciniumfruchtextrakt keinen oder nur geringfügigen Abbaureaktionen unterliegen.

Im Sinne dieser Erfindung ist ein Hefeextrakt oder Hefezell-Lysat ein Lysat von Hefezellen, insbesondere wie unten weiter beschrieben, der nicht-wasserlösliche als auch wasserlösliche Bestandteile umfasst. Die wasserlöslichen Bestandteile sind dabei gekennzeichnet durch ihre Löslichkeit in Wasser. Sie treten während der Autolyse aus der Zelle aus und liegen überwiegend im wässrigen Medium vor, welches die Zellen umgibt. Bei den wasserlöslichen Bestandteilen handelt es sich meist um ein Gemisch aus wasserlöslichen Proteinen, Peptiden, freien Aminosäuren, Kohlenhydraten, Nukleinsäureabbauprodukten, organischen Säuren sowie den Lipidabbauprodukten. wasserlösliche Bestandteile sind insbesondere:
- die freien Aminosäuren Threonin, Serin, Asparagin, Glutaminsäure, Prolin, Cystein, Glycin, Alanin, Citrulin, Valin, Cystin, Methionin, Isoleucin, Leucin, Tyrosin, Phenylalanin, Homocystin, Ornithin, Lysin, Histidin, Arginin und die daraus gebildeten Dipeptide, Oligopeptide (3-10 Aminosäurerestmoleküle) und Polypeptide (11-100 Aminosäurerestmoleküle) sowie wasserlösliche Proteine (mehr als 100 Aminosäurerestmoleküle);
- Glucose und daraus aufgebaute wasserlösliche Di-, Tri-, Oligo- und Polysaccharide;
- Nucleoside und Nukleotide wie Adenosinmonophosphat, Guanosinmonophosphat, Uracilmonophosphat;
- Propionsäure, Bernsteinsäure, Maleinsäure, Ameisensäure, Essigsäure, Oxalsäure, Zitronensäure, Weinsäure, und Milchsäure sowie
- Glycerin.

Die nicht wasserlöslichen Bestandteile bestehen aus im Wesentlichen intakten Zellwänden und weiteren Zellbestandteilen, die nach der Autolyse nicht in einer wasserlöslichen Form vorliegen. Zu diesen weiteren, nicht wasserlöslichen Bestandteilen gehören insbesondere Fragmente der Zellmembran, des Zellkerns, der RNA und DNA, der Mitochondrien sowie Lipide und die wasserunlöslichen Abbauprodukte der Lipide.

Vorzugsweise umfasst ein erfindungsgemäßes Verkapselungsprodukt:
a) 0,5 - 50,0 Gew.-% Vacciniumfruchtextrakt,
b) 30 - 98,9 Gew.-% Hefezell-Lysat (umfassend einen Anteil nicht-wasserlöslicher Bestandteile eines Hefezell-Lysats und einen Anteil wasserlöslicher Bestandteile eines Hefezell-Lysats),
c) 0,1 - 10,0 Gew.-% Natriumchlorid,
d) 0,1 - 10,0 Gew.-% Restwasser.

Bevorzugt umfasst oder besteht das erfindungsgemäße Verkapselungsprodukt aus:
a) 10,0 - 30,0 Gew.-% Vacciniumfruchtextrakt,
b) 51,0 - 89,4 Gew.-% Hefezell-Lysat
c) 0,5 - 9,0 Gew.-% Natriumchlorid
d) 0,1 - 10,0 Gew.-% Restwasser.

Besonders bevorzugt umfasst oder besteht das erfindungsgemäße Verkapselungsprodukt aus:
a) 15,0 - 25,0 Gew.-% Vacciniumfruchtextrakt,
b) 57,0 - 84,4 Gew.-% Hefezell-Lysat
c) 0,5 - 8,0 Gew.-% Natriumchlorid
d) 0,1 - 10,0 Gew.-% Restwasser.

Der Begriff Vacciniumfruchtextrakt umfasst den meist gefärbten, überwiegend wasserlöslichen Extrakt aus den Früchten (Beeren) der amerikanischen Heidelbeere oder Kulturheidelbeere (*Vaccinium corymbosum*), Preiselbeere (*Vaccinium vitis-idaea*), gewöhnliche Moosbeere (*Vaccinium oxycoccos*), großfruchtige Moosbeere oder Kranbeere (cranberry) (*Vaccinium macrocarpon*), kleinfrüchtige Moosbeere (*Vaccinium microcarpum*), Alpen Rauschbeere (*Vaccinium gaulthe*roides), Rauschbeere (*Vaccinium uliginosum*), Big Huckleberry (*Vaccinium membranaceum*), Red Huckleberry (*Vaccinium parvifolium*), Sparkleberry (Vac*cinium arboreum*), Ohelo-Beere (*Vaccinium reticulatum*), und der kanadischen Blaubeere (*Vaccinium myrtilloides*). Im Rahmen der vorliegenden Erfindung ist der Extrakt der Heidelbeere (Bilberry) besonders bevorzugt.

Die Fett- bzw. Öllöslichkeit dieser Extrakte ist nicht bzw. nur äußerst beschränkt vorhanden. Zu den wertgebenden Inhaltsstoffen zählen die Anthocyane, die einen Anteil von vorzugsweise 0,1-30 Gew.-% des trockenen Extraktes ausmachen. Der Anteil der Anthocyane an der erfindungsgemäßen Zubereitung beträgt vorzugsweise 0,125-12,5 Gew.-%, bevorzugt 2,5-7,5 Gew.-% und besonders bevorzugt 3,75-6,25 Gew.-%. Die in der Gattung Vaccinium vorkommenden Anthocyane Cyanidin, Delphinidin, Malvidin, Petunidin und Peonidin liegen überwiegend glycosidisch gebunden als Cyanidin-3-arabinosid, Cyanidin-3-galactosid, Cyanidin-3-glucosid, Delphinidin-3-arabinosid, Delphinidin-3-galactosid, Delphinidin-3-glucosid, Malvidin-3-arabinosid, Malvidin-3-galactosid, Malvidin-3-glucosid, Petunidin-3-arabinosid, Petunidin-3- galactosid, Petunidin-3-glucosid, Peonidin-3- arabinosid, Peonidin-3-galactosid, Peonidin-3-glucosid vor. Als weitere Inhaltstoffe des Extraktes sind vor allem Saccharide, organische Säuren, und weitere Polyphenole, wie Flavonoide und Gerbsäuren (Tannine) sowie Vitamine zu nennen.

Der Vacciniumfrüchteextrakt kann durch an sich bekannte Extraktionsverfahren aus den Früchten (Beeren) der Pflanzen gewonnen werden. Hierzu zählen z.B. die Mazeration oder Perkolation. Als Extraktionsmedium kann z.B Wasser und Ethanol bzw. Mischungen daraus verwendet werden. Anstelle von Ethanol können auch Methanol und andere wasserlösliche Lösungsmittel verwendet werden. Die Temperaturwahl und mechanische Desintegration der Früchte können die Extraktion unterstützen. Nach dem Stand der Technik empfiehlt sich auch die mechanische Desintegration der Früchte z.B. durch Rührer, Homogenisatoren oder Ultraschall. Ferner können weitere extraktionsfördernde Stoffe, wie Säuren, Basen und Enzyme eingesetzt werden. Der Extrakt kann ohne weitere Behandlung, eingedickt oder getrocknet der Suspension der lysierten Hefe hinzugefügt werden.

Die Hefen werden bevorzugt ausgewählt aus den Ascomycetes (Schlauchpilzen), wobei die Familie Saccharomycetaceae, die Unterfamilie Saccharomycetoideae und die Gattung Saccharomyces oder die Familie Cryptococcaceae und deren Gattungen Candida, Torulopsis und Kloeckera bevorzugt werden. Beispiele hierfür sind Saccharomyces Carlsbergensis, Saccharomyces Bayanus und Saccharomyces Uvarum. Wegen sehr guter Verfügbarkeit und der Herstellung in technisch relevanten Mengen, kommt vor allem die Verwendung von Saccharomyces Cerevisiae (auch bekannt als Bäckereihefe, Bierhefe) und Candida Utilis (auch Torula Hefe, früher Torulopsis utilis oder Torula utilis) in Betracht.

Autolysierte Hefe ist im Sinne dieser Erfindung das Produkt der unvollständigen Hefeautolyse. Die Hefeautolyse ist im Stand der Technik bekannt und bezeichnet den Abbau der Hefen durch die eigenen Enzyme. Sie wird durchgeführt, indem eine wässrige Hefesuspension bei einer Temperatur zwischen ca. 40 und 65°C für einen Zeitraum von etwa einer Stunde bis zu etwa 4 Tagen sich selbst überlassen wird. Dabei bewirken die zelleigenen Enzyme einen Abbau und ermöglichen die Lösung der Zelleinhaltsstoffe. Die Autolyse kann durch die Verwendung von Plasmolysern, Säuren, Basen, nicht zelleigenen Enzymen und organischen Lösungsmitteln unterstützt werden. Die Verwendung dieser Hilfsstoffe wird ebenfalls im Stand der Technik beschrieben. Insbesondere hervorzuheben ist die Verwendung von Natriumchlorid (Kochsalz) als Plasmolyser. Es wird zu Beginn der Hefesuspension zugesetzt und bewirkt eine Diffusion des zellinneren Wassers durch die zu diesem Zeitpunkt noch intakte, semipermeable Zellmembran.

Im Verlauf der Autolyse wird die Zellmembran so beschädigt, dass die gelösten Zellinhaltsstoffe in das umgebende wässrige Medium übergehen können. Allerdings ist der vollständige Abbau (vollständige Autolyse) der Zellen nicht das erfindungsgemäße Ziel. Vielmehr ist es ein ausdrücklicher Bestandteil der Erfindung, dass die Zelle nur zum Teil (unvollständig) aufgelöst wird und unlösliche Zellreste sowie lösliche Bestandteile als Produkt der Autolyse entstehen. Ein wichtiger Bestandteil der unlöslichen Zellereste sind im Wesentlichen intakte Zellwände, die die restlichen ungelösten Bestandteile umschließen. Sowohl die unlöslichen Zellreste als auch die wasserlöslichen Bestandteile können erfindungsgemäß in dem Verkapselungsprodukt verwendet werden. Das Verhältnis aus nicht-wasserlöslichen Bestandteilen zu löslichen Bestandteilen beträgt vorzugsweise 0,1 : 1 bis 9 : 1, insbesondere bevorzugt 0,4 : 1 bis 2,3 : 1 und besonders bevorzugt 0,7 : 1 bis 1,5 : 1.

Zur erfindungsgemäßen Eigenschaft des Produkts gehört die ausgewogene Freisetzung der Vacciniumfruchtextrakte im Magen und im Darm. Unter einer ausgewogenen Freisetzung im Magen und im Darm wird im Sinne dieser Erfindung ein Verhältnis der Magenfreisetzung zur Darmfreisetzung von 0,3 : 1 bis 3 : 1, bevorzugt 0,4 : 1 bis 2,5 : 1 und besonders bevorzugt von 0,5 :1 bis 2:1 verstanden. Die Magen bzw. Darmfreisetzung wird nach folgendem in vitro Verdauungstest gemäß Analyst, 2002, 127, 1638-1641: Development of a sequential enzymolysis approach for the evaluation of the bioaccessibility of Cd and Pb from cocoa durchgeführt:

Als Verdauungssäfte werden folgende Lösungen verwendet:

| "Kunstspeichel" | |
|---|---|
| 5,0 g | Kaliumhydrogencarbonat |
| 0,64 g | Kaliumchlorid |
| 0,15 g | Natriumcarbonat |
| 20,61 mg | α-Amylase |
| 100 mg | Mucin |
| in 1000 ml | dest. Wasser |
| pH-Wert mit NaOH auf 6,9 einstellen | |

| "Magensaft" | |
|---|---|
| 1 g | Pepsin |
| 0,88 g | Natriumchlorid |
| in 100 ml | dest. Wasser |
| pH-Wert mit HCl auf 2,5 einstellen | |

| "Darmsaft" | |
|---|---|
| 30 mg | KCI |
| 50 mg | CaCl₂ |
| 20 mg | MgCl₂ |
| 20 mg | NaHCO₃ |
| 30 mg | Trypsin |
| 900 mg | Pankreatin |
| 900 mg | Galle, lyophilisiert |
| in 100 ml d | est. Wasser |
| pH-Wert mit NaOH auf 6,9 einstellen | |

1 g des Verkapselungsprodukts werden mit 6,25 g Kunstspeichel unter ständigem Rühren versetzt und 15 min bei 37°C inkubiert. Der pH-Wert der Lösung wird mit verdünnter Salzsäure auf 2,5 eingestellt. Dazu werden 20 ml des "künstlichen Magensaftes" zugegeben und unter Rühren bei 37°C 4 h lang inkubiert. Zur Bestimmung der Freisetzung der Anthocyane im Magen werden aus dieser Lösung 5 g entnommen, und mittels RP-HPLC mit UV-Detektion bestimmt. Nachdem der pH-Wert auf 6,9 eingestellt wurde, werden 10 ml des "Darmsaftes" zugegeben und die Mischung unter Rühren weitere 3 h bei 37°C inkubiert. Anschließend wird die enzymatische Reaktion gestoppt, indem der pH-Wert auf pH 2,5 mit verdünnter Salzsäure eingestellt wird. 5 g der so eingestellten Probe werden zur Bestimmung der Darmfreisetzung per RP-HPLC verwendet.

Zum Vergleich bzw. zur quantitativen Auswertung der Freisetzung von Anthocyanen aus dem eingekapselten Bilberry-Extrakt während des Verdauungsvorganges *in vitro* wurde eine 20%-ige wässrige Lösung des unverkapselten Bilberry-Extraktes als Standard mittels RP-HPLC mit UV-Detektion vermessen. Unter der Annahme dass diese wässrige Lösung einer 100%-igen Freisetzung im Magen bzw. im Darm entspricht, wurde die Freisetzung der Anthocyane aus dem verkapselten Bilberry-Extrakt in der Magen-Darm-Passage berechnet (siehe Tabelle 1).

Die Erfindung betrifft ferner ein Verfahren zur Herstellung eines Verkapselungsproduktes umfassend:
a) Bereitstellen eines Vacciniumfruchtextrakts,
b) Bereitstellen eines Anteils nicht-wasserlöslicher Bestandteile und eines Anteil wasserlöslicher Bestandteile eines Hefezell-Lysats, und
c) Mischen des Vacciniumfruchtextrakts mit den Hefezell-Lysatanteilen und Wasser bei einer Temperatur zwischen 0 und 60°C für einen Zeitraum von weniger als 4 Stunden, vorzugsweise zwischen 6 und 30 min.

Optional wird die in c) hergestellte Mischung getrocknet.

Die in Schritt c) hergestellte Mischung umfasst oder besteht vorzugsweise aus:
a) 0,2 - 17,0 Gew.-% Vacciniumfruchtextrakt,
b) 10,0 - 33,0 Gew.-% Hefezell-Lysat (umfassend einen Anteil nicht-wasserlöslicher Bestandteile eines Hefezell-Lysats und einen Anteil wasserlöslicher Bestandteile eines Hefezell-Lysats),
c) 0,1 - 3,0 Gew.-% Natriumchlorid,
d) 47,0 - 89,7 Gew.-% Wasser.

Die in Schritt c) hergestellte Mischung umfasst oder besteht bevorzugt aus:
a) 3,0 - 10,0 Gew.-% Vacciniumfruchtextrakt,
b) 17,0 - 30,0 Gew.-% Hefezell-Lysat
c) 0,2 - 3,0 Gew.-% Natriumchlorid
d) 57,0 - 79,8 Gew.-% Wasser.

Die in Schritt c) hergestellte Mischung umfasst oder besteht besonders bevorzugt aus:
a) 5,0 - 8,0 Gew.-% Vacciniumfruchtextrakt,
b) 19,0 - 28,0 Gew.-% Hefezell-Lysat
c) 0,2 - 3,0 Gew.-% Natriumchlorid
d) 61,0 - 75,8 Gew.-% Wasser.

Das Mischen des Ansatzes kann mit an sich bekannten Ausrüstungen, wie Rührern, Rotor-Stator-Dispergatoren bzw. Hochdruckhomogenisatoren erfolgen. Bevorzugt beträgt die Mischzeit zwischen 6 und 20 min und besonders bevorzugt zwischen 6 und 15 min. Das Mischen findet bevorzugt bei Temperaturen zwischen 10 und 60 °C statt. Besonders bevorzugt findet eine Erwärmung der Mischung von 15 auf 50 °C statt. Das Mischen erfolgt hierbei so lange, bis die - Vacciniumfruchtextrakt reversibel an die wasserlöslichen Bestandteile des Hefezell-Lysats gebunden wird, aber vorzugsweise nicht in das Innere des unlöslichen Zellrests vorgedrungen ist.

Die Trocknung erfolgt durch bekannte Trocknungsverfahren, wie z.B. Sprühtrocknung, Wirbelschichtsprühgranulation, Bandtrocknung, Vakuumtrocknung oder Gefriertrocknung.

Vorzugsweise wird die Sprühtrocknung verwendet, wobei dabei regelmäßig Lufteinlasstemperaturen im Bereich von 120 bis 300 °C, bevorzugt im Bereich von 150 bis 250 °C und besonders bevorzugt im Bereich von 180 bis 220 °C verwendet werden.

Die Luftauslasstemperaturen liegen regelmäßig im Bereich von 50 bis 150 °C, bevorzugt im Bereich von 60 bis 120 °C und besonders bevorzugt im Bereich von 70 bis 100 °C. Dabei können beliebige Zerstäubertypen verwendet werden wie z.B. Düsen, mechanische Zerstäuber und Ultraschallzerstäuber.

Als Düsen können beispielsweise Einstoffdüsen, Druckdüsen, Vollkegeldruckdüsen, Hohlkegeldruckdüsen, Zweistoffdüsen, Dreistoffdüsen und Vierstoffdüsen usw. verwendet werden. Mechanische Zerstäuber (Rotationszerstäuber) umfassen einen rotierenden Körper, der die Zerteilung der Sprühmischung in kleine Tropfen bewirkt. Die rotierenden Körper sind üblicherweise Scheiben oder auch Glocken; ein Beispiel für einen mechanischen Zerstäuber ist ein Scheibenzerstäuber. Geeignete Zerstäuber kann der Fachmann insbesondere je nach Menge der zu trocknenden Sprühmischung und der Größe der erwarteten Aromapartikel auswählen. Als bevorzugte Zerstäuber werden Rotationszerstäuber und insbesondere Scheibenzerstäuber verwendet. Bei der Verwendung der Sprühtrocknung entsteht in Abhängigkeit der verwendeten Ausrüstung und der verwendeten Parameter ein Verkapselungsprodukt mit einer Partikelgröße zwischen 2 und 150 µm. Mit Hilfe der anderen genannten Trocknungsverfahren können sogar auch bis zu 5 mm große Partikel entstehen.

Im Sinne dieser Erfindung können der erfindungsgemäßen Zusammensetzung vor der Trocknung noch weitere Substanzen hinzugefügt werden. Hierzu zählen insbesondere Saccharide einschließlich Stärken, modifizierten Stärken, Maltodextrinen, Dextrosesirup, Stärkeoctenylsuccinat, Saccharose, Dextrose, Gummi Arabicum, Cellulosederivate; sowie Süßstoffe, Proteine und Zuckeralkohole.

Erfindungsgemäße, der Ernährung oder dem Genuss dienende Zubereitungen enthaltend eine oder mehrere erfindungsgemäße Verkapselungsprodukte sind vorzugsweise gewählt aus:

Backwaren (z.B. Brot, Trockenkekse, Kuchen, Muffins, Waffeln, Backmischungen, sonstiges Gebäck), Süßwaren (z.B. weisse, helle oder dunkle Schokoladen, gefüllte Schokoladen (beispielsweise mit aromatisierter Fondant-Masse, Typ After-Eight), Schokoladenriegel, sonstige Riegelprodukte, Kaubonbons, Fruchtgummi, Hart- und Weichkaramellen, Kaugummi, Zuckerperlen, Lutscher), Kapseln (vorzugsweise nahtlose Kapsel zum Direktverzehr, vorzugsweise mit einer Umhüllung basierend auf Gelatine und/oder Alginat), Fettmassen (z.B. Backwarenfüllungen für z.B. Keksfüllungen, Schokoladenfettfüllungen, Riegelfettfüllungen), Aufstreumischungen (Toppings), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke oder Instantpulver (z.B. Instant-Kakao-Getränke, Instant-Tee-Getränke, Instant-Kaffeegetränke, Instant Desserts in Pulverform wie Puddingspulver oder Götterspeise), Fleischprodukte (z.B. Schinken, Frischwurst- oder Rohwurstzubereitungen, gewürzte oder marinierte Frisch- oder Pökelfleischprodukte), Eier oder Eiprodukte (z.B. Trockeneipulver), Getreideprodukte und/oder Nussprodukte (z.B. Frühstückscerealien, Cornflakes, Haferflocken, Schüttmüsli, Müsliriegel, Studentenfutter, süßes Popcorn, Nussriegel, Nuss-Frucht-Riegel, vorgegarte Fertigreis-Produkte), Milchprodukte (z.B. Milchgetränke, Milcheis, Joghurt, Pudding, Kefir, Frischkäse, Weichkäse, Hartkäse, Trockenmilchpulver, Molke, Butter, Buttermilch, teilweise oder ganz hydrolysierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder entsprechende Emulsionen (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Der Gesamtanteil an erfindungsgemäßen Verkapselungsprodukten in einer erfindungsgemäßen der Ernährung oder dem Genuss dienende Zubereitung liegt vorzugsweise im Bereich von 0,1 bis 10 Gew.-%, bevorzugt im Bereich von 0,25 bis 5 Gew.-%, und besonders bevorzugt im Bereich von 0,5 bis 3 Gew.-%, jeweils bezogen auf die Gesamtmasse der Zubereitung.

Erfindungsgemäße der Ernährung oder dem Genuss dienende Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Bevorzugte erfindungsgemäße, der Ernährung oder dem Genuss dienende Zubereitungen sind:

Süßwaren wie z.B. Lutschbonbons, Kaugummis, Fruchtgummis, Kaubonbons, (Erfrischungs)Dragees, Komprimate, Hartkaramellen, Schokocremes, Konfekt und Schokolade, Backwaren wie Kuchen, Waffeln und Kekse, Snacks, Instant-Mahlzeiten sowie anderen Instant-Produkten (Suppen, Saucen, Getränkepulver und -granulate, Würzmischungen), Speiseeis, Fruchtzubereitungen (Marmelade, Konfitüre, Fruchtsaucen), Desserts (Puddings, Götterspeise), Milchprodukte (Quark, Joghurt, Milchdrinks, Molkezubereitungen) sowie Cerealien (Cornflakes, Müsliriegel). Daneben ist auch die Verwendung in Nahrungsergänzungsmitteln und pharmazeutischen Produkten, wie Lutschtabletten, Hals- oder Hustenbonbons, pharmazeutischen Pulvern, Tabletten oder Granulaten vorteilhaft.

Bei erfindungsgemäßen Snacks handelt es sich meist um salzige Knabberartikel, wie beispielsweise Kartoffel-Maischips, Extrudate, Pellets, Popcorn, Cracker, Laugengebäck und in Fett oder im Ofen ausgebackene Teigprodukte. Erfindungsgemäße Verkapselungsprodukte oder eine die erfindungsgemäßen Verkapselungsprodukte umfassende Aromakomposition können in einen Snack-Artikel eingearbeitet oder auf diesen aufgebracht werden Die Einarbeitung bzw. Aufbringung kann über Aufstreuwürze, aufgesprühte Öl-Slurry, Fettfüllung oder Teigaromatisierung erfolgen.

Als weitere Bestandteile für erfindungsgemäße, der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse-oder Fruchtsäfte oder -pasten oder deren Gemische), verdauliche oder nicht verdauliche Kohlenhydrate (z.B. Amylose, Amylopektin, Inulin, Xylane, Cellulose), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, Kühlwirkstoffe wie z.B. Menthol, Mentholderivate (z.B. L-Menthol, L-Menthyllactat, L-Menthylglutarat, L-Menthylsuccinat) oder Cubebol, synthetische, natürliche oder naturidentische Aromastoffe oder Riechstoffe sowie Geruchskorrigentien.

Erfindungsgemäße, der Ernährung oder dem Genuss dienende Zubereitungen können zusätzlich ein oder mehrere Geschmackskorrigenzien enthalten, vorzugsweise gewählt aus der folgenden Liste: Nucleotide (z.B. Adenosin-5'-monophosphat, Cytidin-5'-monophosphat) oder deren pharmazeutisch akzeptable Salze, Lactisole, Natriumsalze (z.B. Natriumchlorid, Natriumlactat, Natriumcitrat, Natriumacetat, Natriumgluconoat), weitere Hydroxyflavanone (z.B. Eriodictyol, Homoeriodictyol oder deren Natriumsalze), insbesondere gemäß US 2002/0188019, Hydroxybenzoesäureamide nach DE 10 2004 041 496 (z.B. 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-N-(4-hydroxy-3-methoxybenzyl)amid, 2-Hydroxy-benzoesäure-N-4-(hydroxy-3-methoxybenzyl)amid , 4-Hydroxybenzoesäure-N-(4-hydroxy-3-methoxybenzyl)amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)ethylamid, 2,4-Dihydroxybenzoesäure-N-(4-hydroxy-3-ethoxybenzyl)amid, 2,4-Dihydroxybenzoesäure-N-(3,4-dihydroxybenzyl)amid und 2-Hydroxy-5-methoxy-N-[2-(4-hydroxy-3-methoxyphenyl)ethyl]amid (Aduncamid), 4-Hydroxybenzoesäurevanillylamid), bittermaskierende Hydroxydeoxybenzoine gemäß WO 2006/106023 sowie den darauf basierenden Dokumenten (Symrise) (z.B. 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon), Aminosäuren (z.B. gamma-Aminobuttersäure nach WO 2005/096841 zur Verminderung oder Maskierung eines unangenehmen Geschmackseindrucks wie Bitterkeit), Äpfelsäureglycoside nach WO 2006/003107, salzig schmeckende Mischungen nach WO 2007/045566, Diacetyltrimere nach WO 2006/058893, Divanillin, Gemische von Molkeproteinen mit Lecithinen und/oder bittermaskierende Substanzen wie Gingerdione gemäß WO 2007/003527.

Erfindungsgemäße, der Ernährung oder dem Genuss dienende Zubereitungen können zusätzlich ein oder mehrere Alkamide enthalten, vorzugsweise ausgewählt aus der Gruppe bestehend aus: 2E,4E-Decadiensäure-N-isobutylamid (Pellitorin), 2E,4Z- Decadiensäure-N-isobutylamid (cis-Pellitorin), 2Z,4Z- Decadiensäure-N-isobutylamid, 2Z,4E- Decadiensäure-N-isobutylamid, 2E,4E-Decadiensäure-N-([2S]-2-methylbutyl)amid, 2E,4E-Decadiensäure-N-([2S]-2-methylbutyl)amid, 2E,4E-Decadiensäure-N-([2R]-2-methylbutylamid), 2E,4Z-Decadiensäure-N-(2-methylbutyl)amid, 2E,4E-Decadiensäure-N-piperid (Achilleamid), 2E,4E-Decadiensäure-N-piperid (Sarmentin), 2E-Decensäure-N-isobutylamid, 3E-Decensäure-N-isobutylamid, 3E-Nonensäure-N-isobutylamid, 2E,6Z,8E-Decatriensäure-N-isobutylamid (Spilanthol), 2E,6Z,8E-Decatriensäure-N-([2S]-2-methylbutyl)amid (Homospilanthol), 2E,6Z,8E-Decatriensäure-N-([2R]-2-methylbutyl)amid, 2E-Decen-4-insäure-N-isobutylamid, 2Z-Decen-4-insäure-N-isobutylamid, Sanshoole.

Obwohl die Freisetzungscharakteristik der erfindungsgemäßen Verkapselungsprodukte auf die Freisetzung im Verdauungstrakt zugeschnitten ist, können diese Bestandteil von Produkten sein, die nicht zum Schlucken und Verdauen durch den Menschen vorgesehen sind, z.B. von Bedarfsgegenständen wie Körperpflegeprodukten, Haushaltsprodukten, Tabakwaren (z.B. Zigaretten), Kosmetikprodukten (Gesichtsmasken) und von Mundpflegeprodukten wie Zahnpasten, Zahngelen, Zahncremes, Zahnpflegekaugummis und Mundwassern.

Die Erfindung wird anhand der nachfolgenden Beispiele exemplarisch dargelegt. Sofern nicht anders angegeben beziehen sich alle Angaben auf das Gewicht.

### Beispiel 1: Erfindungsgemäßes Verkapselungsprodukt

300 g frische Backhefe (Vital Gold von Fa. Deutsche Hefewerke GmbH Nürnberg) und 10 g Kochsalz (Natriumchlorid) werden in einem Becherglas mit einem Kochlöffel vermischt. Durch die Plasmolyse geht die Hefemasse in eine relativ niedrigviskose Suspension über. Die Suspension wird in ein Wasserbad mit einer Temperatur von 60°C gestellt, für 3 Stunden abgedeckt stehengelassen und von Zeit zu Zeit mit einem Löffel umgerührt. Hierbei findet die Autolyse statt und aus der anfangs lebenden, intakten Hefe entstehen die unlöslichen Zellreste sowie die wasserlöslichen Bestandteile des Hefezell-Lysats. Der Ansatz wird im kalten Wasserbad auf 20°C abgekühlt. 37,5 g Heidelbeerextrakt (Bilberry - Extrakt von Fa. Kaden Biochemicals GmbH, Hamburg, Deutschland) werden hinzugefügt und 10 min mit einem Ultra Turrax dispergiert. Die Suspension wird einem Sprühturm Niro Minor mit einem Scheibenzerstäuber zugeführt. Die Lufteinlasstemperatur beträgt 200°C und die Luftauslasstemperatur beträgt 80°C.

### Beispiel 2: Erfindungsgemäßes Verkapselungsprodukt

500 g Springer (R) 2000 (getrocknete autolysierte Hefe einschließlich unlöslicher Zellreste und löslichem Hefeextrakt von Fa. Bio Springer, Maisons-Alfort Cedex, Frankreich) werden in 1500 g Wasser suspendiert. 125 g Heidelbeerextrakt (Bilberry - Extrakt von Fa. Kaden Biochemicals GmbH, Hamburg, Deutschland) werden hinzugefügt. Mit einem Ultra-Turrax wird der Ansatz für 10 min bei 3000 U/min dispergiert. Er erwärmt sich dabei von 18 auf 49°C. Die Suspension wird einem Sprühturm Niro Minor mit einem Scheibenzerstäuber zugeführt. Die Lufteinlasstemperatur beträgt 200°C und die Luftauslasstemperatur beträgt 80°C. Es entsteht das erfindungsgemäße Verkapselungsprodukt mit einem Heidelbeerextraktgehalt von 20% und einer Partikelgröße von 12µm.

Bei der Betrachtung unter dem Mikroskop bei 1200-facher Vergrößerung (nach Suspendierung in destilliertem Wasser) ist der unlösliche Zellrest mit der im Wesentlichen intakten Zellwand erkennbar. Sie grenzt die Reste der nahezu ungefärbten übrigen Zellsubstanz ein. Dies zeigt an, dass die übrige Zellsubstanz im Zellinneren keine wesentliche Verkapselungsfunktion ausführt. Rot-bläulich gefärbt erscheint lediglich die Zellwand durch die Anlagerung des Heidelbeerextraktes in Kombination mit dem löslichen Hefeextrakt (wasserlösliche Bestandteile des Hefezell-Lysats).

### Beispiel 3 (Vergleichsbeispiel, nicht erfindungsgemäß)

500 g Springer (R) 2000 aus Beispiel 2 werden in 1500 g Wasser (20°C) suspendiert. Die unlöslichen Zellreste werden von den wasserlöslichen - Bestandteilen mittels Filtration durch einen Papierfilter getrennt und bei 40°C in einem Umlufttrockenschrank getrocknet. Der so erhaltene lösliche Hefeextrakt (Filterdurchgang) wird ebenfalls bei 40°C im Umlufttrockenschrank getrocknet, wobei die Weiterverarbeitung in Beispiel 4 beschrieben wird.

Die unlöslichen Zellreste (192 g ) wurden in 576 g Wasser suspendiert, 48 g Heidelbeerextrakt aus Beispiel 1 hinzugefügt und 10 min mit einem Ultra Turrax bei 3000 U/min dispergiert. Die Trocknung erfolgte wie in Beispiel 1.

Bei der Betrachtung unter dem Mikroskop bei 1200-facher Vergrößerung ist der unlösliche Zellrest mit der im Wesentlichen intakten Zellwand erkennbar. Sie grenzt die Reste der rot-bläulich gefärbten übrigen Zellsubstanz ein. Dies zeigt an, dass die Verkapselungsfunktion hauptsächlich durch die übrige Zellsubstanz im Zellinneren ausgeübt wird.

### Beispiel 4 (Vergleichsbeispiel, nicht erfindungsgemäß)

Der lösliche Hefeextrakt (296 g) aus Beispiel 3 wurde in 888 g Wasser aufgelöst und anschließend mit 74 g des Heidelbeerextraktes aus Beispiel 1 versetzt. Die Behandlung mit dem Ultra-Turrax und die Trocknung werden wie in Beispiel 1 durchgeführt.

### Beispiel 5: Verdauungstest

Die Beispiele 2 bis 4 wurden in dem weiter oben beschriebenen in vitro Verdauungstest eingesetzt und die Ergebnisse in der Tabelle dargestellt.

**Tabelle 1: in vitro Freisetzung der Anthocyane nach dem Verdauungstest**

| Beispiel | Beschreibung | Freisetzung von Anthocyanen im Magen [%]* | Freisetzung von Anthocyanen im Darm [%]* |
|---|---|---|---|
| 2 | erfindungsgemäß | 21,97 | 23,9 |
| 3 | Vergleich | 25,7 | 5,2 |
| 4 | Vergleich | 30,5 | 11,2 |

| | | | |
|---|---|---|---|
| * bezogen auf den Gesamtgehalt der Anthocyane im Verkapselungsprodukt | | | |

Wie der Tabelle 1 zu entnehmen ist, werden die Anthocyane bei allen getesteten Verkapselungsformen sehr gut im Magen freigesetzt. Im erfindungsgemäßen Beispiel 2 ist jedoch bereits zu erkennen, dass die Freisetzung im Magen in geringerem Ausmaß stattfindet als in den vergleichenden Beispielen 3 und 4. Dies ist unerwartet, da die Zusammensetzung des erfindungsgemäßen Beispiels 2 eigentlich nur eine Mischung aus den Beispielen 3 und 4 ist. Demnach wäre in Beispiel 2 eine Freisetzung im Magen zwischen 25,7 und 30,5 % zu erwarten gewesen.

Die Freisetzung im Darm ist mit 23,9 % beim erfindungsgemäßen Beispiel am höchsten. Auch diese Charakteristik ist unerwartet, da eigentlich eine Freisetzung zwischen 5,2 und 11,2 % zu erwarten gewesen wäre.

### Beispiel 6: Sprühgetrocknete erfindunqsgemäße Verkapselungsprodukte mit weiteren Trägerstoffen

| | Zubereitung (Einsatz in Gew.-%) | | | |
|---|---|---|---|---|
| Inhaltsstoff | A | B | C | D |
| Trinkwasser | 65% | 65% | 65% | 65% |
| Maltodextrin mit Dextroseäquivalent 20 | 8% | 0% | 4% | 6% |
| Gummi Arabicum | 0% | 8% | 4% | 2% |
| Hefe Springer (R) 2000 | 20% | 20% | 20% | 20% |
| Heidelbeerextrakt | 7% | 7% | 7% | 7% |

Die jeweilige Zubereitung wird in ein geeignetes Mischgefäß gegeben und für 10 min mit einem Ultra-Turrax bei 3000 U/min gemischt. Es wird wie in Beispiel 2 angegeben weiter verfahren. Es entsteht das erfindungsgemäße Produkt mit Anteilen weiterer Trägerstoffe.

### Beispiel F1: Kaugummis mit erfindungsgemäßen Verkapseiungsprodukten

Die Kaugummibase K2 bestand aus 28,5% Terpenharz, 33,9% Polyvinylacetat (MW = 14000), 16,25% hydriertes Pflanzenöl, 5,5% Mono- und Diglyceride, 0,5% Polyisobuten (MW 75000), 2,0% Butyl Rubber (Isobuten-Isopren-Copolymer), 4,6% amorphes Siliciumdioxid (Wassergehalt ca. 2,5%), 0,05% Antioxidans tert.-Butylhydroxytoluol (BHT), 0,2% Lecithin, und 8,5% Calciumcarbonat. Die Herstellung der Kaugummibase K2 und der Kaugummis kann analog zu US 6,986,907 erfolgen.

| | I (Gew.-%) | II (Gew.-%) | III (Gew.-%) |
|---|---|---|---|
| Kaugummibase K2 | 25,30 | 27,30 | 26,30 |
| Sorbitol | Ad 100 | Ad 100 | Ad 100 |
| Glycerin | 2,40 | 2,40 | 2,40 |
| Lecithin | 7,00 | 7,00 | 7,00 |
| Aspartam | 0,14 | 0,14 | 0,14 |
| Verkapseltes Aspartam | 0,68 | 0,68 | 0,48 |
| Menthol, sprühgetrocknet | 1,00 | 0,50 | 0,40 |
| Kirscharoma, sprühgetrocknet | - | 1,20 | - |
| Citronen-Aroma | 1,10 | 1,30 | 1,68 |
| Orangenöl, natürlich | 0,40 | - | - |
| Verkapselungsprodukt aus Beispiel 1 | 1,45 | - | 0,50 |
| Verkapselungsprodukt aus Beispiel 2 | - | 1,15 | 0,50 |

Die Kaugummis der Rezeptur (I) wurden als Streifen, die der Rezepturen (II) und (III) als Pellets ausgeformt.

### Beispiel F2: Zuckerfreie Hartkaramellen mit erfindungsgemäßen Verkapselungsprodukten

| ***Inhaltsstoff*** | A (Gew.-%) | B (Gew.-%) |
|---|---|---|
| Palatinit, Typ M | Ad 100 % | Ad 100 % |
| Wasser | 24,82 % | 24,82 % |
| Pfefferminzaroma | 0,15 % | 0,05 % |
| Orangenaroma | - | 0,10 % |
| Hesperetin | - | 0,01 % |
| Spilanthol | - | 0,01 % |
| Trans-Pellitorin | 0,01 % | - |
| Verkapselungsprodukt aus Beispiel 2 | 0,75 % | 0,50 % |

Palatinit wurde mit Wasser gemischt und die Mischung bei 165 °C aufgeschmolzen und anschließend auf 115°C abgekühlt. Aroma und erfindungsgemäßes Verkapselungsprodukt, sowie trans-Pellitorin im Falle A und Spilanthol und Hesperetin im Falle B, wurden zugegeben und nach dem Durchmischen in Formen gegossen, nach dem Erstarren aus den Formen entfernt und anschließend einzeln verpackt.

### Beispiel F3: Fettarme Joghurts mit erfindungsgemäßen Verkapselungsprodukten

| | Zubereitung (Angaben in Gew.-%) | | |
|---|---|---|---|
| ***Inhaltsstoff*** | A | B | C |
| Sucrose | 10 % | 8 % | 6 % |
| Tagatose | - | - | 0,5 % |
| Fructose | - | - | 0,5 % |
| Hesperetin | - | 0,01 % | 0,005 % |
| Phloretin | - | - | 0,005 % |
| Pfirsicharoma | 0,30 % | - | 0,40 % |
| Erdbeer-Rhabarberaroma | - | 0,25% | - |
| Verkapselungsprodukt aus Beispiel 1 | 0,25 % | 0,90 % | 0,40 % |
| Verkapselungsprodukt aus Beispiel 2 | 0,40 % | - | 0,50 % |
| Joghurt, 0,1 % Fett | Ad 100 % | Ad 100 % | Ad 100 % |

Die Inhaltsstoffe wurden gemischt und bei 5°C gekühlt.

### Beispiel F4: Fettarme Joghurts mit erfindungsgemäßen Verkapselungsprodukten

| | Zubereitung (Angaben in Gew.-%) | | |
|---|---|---|---|
| ***Inhaltsstoff*** | A | B | C |
| D-Tagatose | 0,482 % | 0,482 % | 0,482 % |
| Sucralose | 0,003 % | 0,003 % | 0,003 % |
| Aspartam | 0,005 % | 0,005 % | 0,005 % |
| Acesulfam K | 0,01 % | 0,01 % | 0,01 % |
| Hesperetin | - | 0,01 % | 0,005 % |
| Phloretin | - | - | 0,005 % |
| Himbeer-Zitronenaroma | 0,30 % | - | 0,40 % |
| Erdbeer-Rhabarberaroma | - | 0,25% | - |
| Verkapselungsprodukt aus Beispiel 1 | 0,25 % | 0,90 % | 0,40 % |
| Verkapselungsprodukt aus Beispiel 2 | 0,40 % | - | 0,50 % |
| Joghurt, 0,1 % Fett | zu 100 % auffüllen | zu 100 % auffüllen | zu 100 % auffüllen |

Die Inhaltsstoffe wurden gemischt und bei 5°C gekühlt.

### Beispiel F5: Diätschokolade auf Basis von Fructose mit erfindungsgemäßen Verkapselungsprodukten

Es wurde eine für Diabetiker geeignete Schokolade aus folgenden Zutaten hergestellt und in rechteckige Tafeln gegossen:
Kakaomasse, Fructose, Magermilchpulver, Kakaobutter, Inulin, Butterreinfett, Emulgator Sojalecithin, Walnüsse, Speisesalz, Joghurt-Vanille-Aroma (enthaltend Vanillin und 1 Gew.-% Hesperetin, bezogen auf das Gesamtgewicht des Vanille-Aromas) und 1 Gew.-% erfindungsgemäßes Verkapselungsprodukt aus Beispiel 1.
Nährwert (pro 100 g):
Eiweiss 8,8 g, Kohlenhydrate 34 g (davon Fructose 23 g, Lactose 7,5 g, Saccharose 1,4 g), Fett 36 g; Ballaststoffe 18,5 (davon 12,2 g Inulin); Natrium: 0,10 g. Kakao-Anteil mindestens 50 Gew.-%.

### Beispiel F6: Müslimischung mit erfindungsgemäßen Verkapselungsprodukten

| Nr. | | A (Gew.-%) | B (Gew.-%) | C (Gew.-%) |
|---|---|---|---|---|
| 1 | Haferflocken | 17,00 | 17,00 | 17,00 |
| 2 | Knusprige Haferflocken Cluster | 10,00 | 10,00 | 10,00 |
| 3 | Reis Crispies | 16,90 | 16,90 | 16,90 |
| 4 | Cornflakes | 16,50 | 16,50 | 16,50 |
| 5 | Korinthen | 3,50 | 3,50 | 3,50 |
| 6 | Haselnüsse, zerhackt | 2,50 | 2,50 | 2,50 |
| 7 | Glucose Sirup aus Weizen, DE 30 | 9,50 | 9,50 | 9,50 |
| 8 | Saccharose | 19,00 | 19,00 | 19,00 |
| 9 | Wasser | 4,00 | 4,00 | 4,00 |
| 10 | Citronensäurepulver, wasserfrei | 0,10 | 0,10 | 0,10 |
| 11 | Verkapselungsprodukt aus Beispiel 1 | 1,00 | - | 0,50 |
| 12 | Verkapselungsprodukt aus Beispiel 2 | - | 1,00 | 0,50 |

Bestandteile Nr. 1 bis 6 in einer Drehtrommel mischen (Mix 1). Bestandteile Nr. 7 bis 9 erwärmen und Bestandteile Nr. 10 bis 12 zugeben (Mix 2). Mix 2 zu Mix 1 geben und gut mischen. Zuletzt die resultierende Müslimischung auf ein Backblech geben und in einem Ofen bei 130°C für 8 Minuten trocknen.

### Beispiel F7: Fruchtgummis mit erfindungsgemäßen Verkapselungsprodukten

| Zutaten: | A (Gew.-%) | B (Gew.-%) |
|---|---|---|
| Wasser | 22,70 | 24,70 |
| Saccharose | 34,50 | 8,20 |
| Glucosesirup, DE 40 | 31,89 | 30,09 |
| Iso Syrup C* Tru Sweet 01750 (Cerestar GmbH) | 1,50 | 2,10 |
| Gelatine 240 Bloom | 8,20 | 9,40 |
| Polydextrose (Litesse^{®} Ultra, Danisco Cultor GmbH) | - | 24,40 |
| Gelber und roter Farbstoff | 0,01 | 0,01 |
| Citronensäure | 0,20 | 0,10 |
| Orangenaroma | - | 0,10 |
| Verkapselungsprodukt aus Beispiel 1 | 1,0 | 0,90 |

### Beispiel F8: Kaubonbon mit erfindungsgemäßen Verkapselungsprodukten

### Zutatenliste:

| | | |
|---|---|---|
| Wasser | | 7,7 % |
| Zucker | Raffinade C4 | 41,0 % |
| Glucose Sirup | Dextrose 40 | 37,3 % |
| gehärtetes Pflanzenfett | Schmelzpunkt 32-36 °C | 6,6 % |
| Lecithin | Emulgator (Sojalecithin) | 0,3 % |
| Gelatine | Schweinegelatine | 0,8 % |
| Fondant | Typ - S30 | 4,9 % |
| Verkapselungsprodukt aus Beispiel 1 | | 1,4 % |

### Beispiel F9: Fruchtiger Müsliriegel mit erfindungsgemäßen Verkapselungsprodukten

### Zutatenliste:

| | | |
|---|---|---|
| Saccharose | Zucker | 16,0 % |
| Glucose Sirup | | 14,0 % |
| Sorbit P 300 | Feuchthaltemittel | 5,0 % |
| Pflanzenfett | | 5,0 % |
| Wasser | | 3,0 % |
| Haferflocken | | 13,3 % |
| Haferflakes | Hafer Extrudate | 10,0 % |
| Cornflakes | | 5,5 % |
| Rice Crispies | Reis Extrudate | 20,0 % |
| Korinthen | | 5,0 % |
| Verkapselungsprodukt aus Beispiel 1 | | 1,3 % |
| Verkapselungsprodukt aus Beispiel 2 | | 1,7 % |
| Zitronensäure, Puder | | 0,2 % |

Beispiel F10: Waffel-Fettfüllung mit erfindungsgemäßen Verkapselungsprodukten

### Zutatenliste:

| | | |
|---|---|---|
| Pflanzenhartfett | Schmelzpunkt 33 - 35 °C | 40,0 % |
| Puderzucker | | 37,0 % |
| Dextrose | Traubenzucker, wasserfrei, mikrofein | 19,0 % |
| Citronensäure | | 0,3 % |
| Verkapselungsprodukt aus Beispiel 1 | | 3,7 % |

Herstellungshinweise: Das Fett auf Raumtemperatur / ca. 21 °C temperieren. Den Puderzucker fein absieben. Alle Zutaten, inklusive Aroma, in einen Hobart-Laborrührer aufschlagen.

### Beispiel F11: Sandkuchen mit erfindungsgemäßen Verkapselungsprodukten

### Zutatenliste / Basisrezeptur Sandkuchen:

| | | |
|---|---|---|
| Weizenmehl | Typ 405 | 16,90 % |
| Weizenstärke | | 4,90 % |
| Saccharose | Zucker, EG Qualität I | 20,20 % |
| Kochsalz | | 0,14 % |
| Kartoffelmehl | | 7,09 % |
| Eigelbpulver | | 1,84 % |
| Backpulver | | 0,70 % |
| Aufschlagemulgator | Mono-Diglyceride | 1,42 % |
| Wasser | | 13,48 % |
| Reinfett | Schmelzpunkt ca. 34 °C | 14,89 % |
| Eier | | 17,02 % |
| beta-Carotin, 1 %ige Lösung | | 1,42 % |

### Herstellungshinweise:

Das Fett temperieren. Alle Trockenstoffe in die Rührschüssel des Hobart-Labormixers geben. Dann Reinfett, Wasser und Eier hinzugeben (Eiergewicht ca. 60 g / Stück). Zuletzt 3 Gew.-% erfindungsgemäßes Verkapselungsprodukt (aus Beispiel 2) zugeben und 1 Minute auf Stufe 1 und 2 Minuten auf Stufe 3 aufschlagen. Den Teig in eine Backform geben und 55 Minuten bei 180° C backen.

### Beispiel F12: Mürbeteigkekse (Industriegualität) mit erfindungsgemäßen Verkapselunqsprodukten

### Zutatenliste:

| | | |
|---|---|---|
| Weizenmehl | Typ 550 | 50,0 % |
| Pflanzenweichfett | Schmelzpunkt 24 / 26 °C | 19,0 % |
| Puderzucker | | 19,0 % |
| Salz | | 0,4 % |
| Ammoniumbicarbonat | Triebmittel | 0,4 % |
| Magermilchpulver | | 1,0 % |
| Maltosesirup | DE 60,5 | 1,2 % |
| Wasser | | 5,50 % |
| Verkapselungsprodukt aus Beispiel 1 | | 3,50 % |

### Herstellungshinweise:

a) Puderzucker, Maltosesirup, Magermilchpulver und Pflanzenweichfett im Hobart-Laborkneter auf Stufe 1 glattlaufen lassen.
b) Mit einem Teil des Wassers das Ammoniumbicarbonat lösen und das restliche Wasser zu a. geben und kurz mischen.
c) Die restlichen Zutaten mit dem Verkapselungsprodukt aus Beispiel 1 zu Mischung a) geben und zu einem glatten Teig ausarbeiten.
d) Den Teig mit der Ausrollmaschine auf ca. 3 mm Dicke ausrollen, evtl. mit einem Reliefholz ein Muster aufbringen, und in der gewünschten Form ausstechen.

Enddicke des Teiges: ca. 2,6 mm; Ofentemperatur: 200°C, Backzeit: 6 Minuten.

### Beispiel F13: Snack-Artikel mit erfindungsgemäßen Verkapselungsprodukten

### Grundrezeptur für die Herstellung von Snacks (Cracker):

Weizenmehl (60-63%), Backpulver (1,0-1,5%), Pflanzenfett (6,0-6,5%), Maltosesirup (2,0-2,5%), Emulgator (1,2-1,8%), Ammoniumbicarbonat (1,5-2,0%), Sprühmagermilchpulver (1,0-1,5%), Backfrischhefe (0,3-0,9%), Speisesalz (0,3-0,6%), Wasser (20,0-23,5%), erfindungsgemäßes Verkapselungsprodukt (0,25 - 2,0 Gew.-%), dabei vorzugsweise ein Verkapselungsprodukt gemäß Beispiel 1 oder 2.

Das Zwiebelaroma ZA der nachfolgenden Rezeptur wurde in einem Anteil von 0,1 bis 2%, bezogen auf die Masse der Cracker-Grundrezeptur, zu der Grundrezeptur zugegeben und die Cracker anschließend gebacken oder frittiert.

### Zwiebelaroma ZA:

2-trans-Hexenal (0,26%), Lauchöl (0,31%), Allinat (0,39%), Methylpropyldisulfid (0,51%), Foetidaöl (0,65%), Dithiazin in Pflanzentriglycerid (1,28%), Dimethyltrisulfid (1,28%), Dipropyldisulfid (2,56%), Dipropyltrisulfid (5,13%), Propylenglykol (8,20%), Zwiebelöl (10,26%), Triacetin (69,17%).

### Beispiel F14: Aufstreuwürzung (Seasoning) mit erfindungsgemäßen Verkapselungsprodukten

Die Herstellung von Aufstreuwürzungen (Seasonings), beispielsweise für Knabberartikel (Snacks), erfolgte nach folgender Rezeptur.

### Grundrezeptur für die Herstellung von Seasonings:

Speisesalz (10-25%), Träger (z.B. Molkenpulver) (40-60%), Füllstoffe (z.B. Fettpulver) (5-15%), Geschmacksverstärker (1,5-3,5%), Hilfsmittel (z.B. Kieselsäure) (0,1-5%), Käsepulver (10-30%), hydrolysierte pflanzliche Proteine (5-10%), HefeExtrakt (5-15%), Gewürze (1-5%), Säuerungsmittel (z.B. Citronensäure) (0,1-1,0%), Farbe (z.B. Paprika-Extrakt) (0,1-1,0%), erfindungsgemäßes Verkapselungsprodukt (1 - 8 Gew.-%), dabei vorzugsweise ein Verkapselungsprodukt gemäß Beispiel 1 oder 2.

### Beispiel F15: Lauchcreme Suppe mit erfindungsgemäßen Verkapselungsprodukten

Die Herstellung einer Lauchcremesuppe erfolgte nach folgender Rezeptur: Milchfettbaustein, Vana Crema (25-30%), Kartoffelstärke (15-25%), Milchzucker, Lactose (18-22%), Maltodextrin (10-12%), Salz (7-9%), Glutamat Mononatriumsalz (2-4%), Pflanzenfett (2-4%), Spinatpulver (1-2%), Zitronensäurepulver (0,2-0,4%), Lauchpulver (1-2%), Lauchstückchen gefriergetrocknet (ca. 10x10 mm) (0,5-1,5%), Gemüsebrühepulver (0,2-0,5%), Curcuma Extrakt (0,05-0,1%), erfindungsgemäßes Verkapselungsprodukt (0,25 - 2,0 Gew.-%), dabei vorzugsweise ein Verkapselungsprodukt gemäß Beispiel 1 oder 2.

Das Laucharoma LA der nachfolgenden Rezeptur wurde in einem Anteil von 0,2 bis 3 Gew.-%, bezogen auf die Masse der Grundrezeptur, zu der Grundrezeptur zugegeben.

### Laucharoma LA:

Propanthiol (0,05%), Diallyldisulfid 1%ig in Pflanzentriglycerid (0,10%), Hexanal (0,15%), Lauchöl (0,20%), Dipropyltrisulfid (0,40%), 2-trans-Hexenal (0,60%), 1-Penten-3-ol (1,20%), Allinat (Allylisothiocyanat) (1,60%), 3-cis-Hexenol (1,71%), Dipropyldisulfid (2,01 %), Pflanzentriglycerid (91,98%).

### Beispiel F16: Würzmischung für Kartoffelchips mit erfindungsgemäßen Verkapselungsprodukten

| Bestandteil | Rezeptur A |
|---|---|
| Natriumglutamat | 3,50 g |
| Käsepulver | 10,00 g |
| Knoblauchpulver | 2,00 g |
| Molkenpulver | 38,86 g |
| Würzextraktöl | 0,20 g |
| Paprikapulver | 9,80 g |
| Kochsalz | 19,00 g |
| Tomatenpulver | 9,00 g |
| Trockenaroma | 2,50 g |
| Siliciumdioxid | 0,02 g |
| Pflanzenöl | 0,02 g |
| Zwiebelpulver | 3,00 g |
| Sahne Aromakonzentrat | 0,03 g |
| Käse Aroma | 0,03 g |
| Tomaten Aromakonzentrat | 0,04 g |
| Verkapselungsprodukt gemäß Beispiel 2 | 2,00 g |

6 g der Würzmischung wurden auf 94 g Kartoffelchips aufgezogen.

### Beispiel F17: Weiße Soße mit erfindungsgemäßen Verkapselungsprodukten

| Bestandteil | Rezeptur A | Rezeptur B |
|---|---|---|
| Maltodextrin | 26,28 g 2 | 26,28 g 2 |
| Kochsalz | 6,25 g | 5,35 g |
| Natriumglutamat | 2,00 g | 2,00 g |
| Pflanzenfett | 5,00 g | 5,00 g |
| Pfeffer, weiss | 0,02 g | 0,02 g |
| Zwiebelpulver | 1,50 g | 1,50 g |
| vorverkleisterte Maisstärke | 30,00 g | 30,00 g |
| Fettpulver | 27,70 g | 27,70 g |
| Verkapselungsprodukt gemäß Beispiel 1 | 1,25 g | - |
| Verkapselungsprodukt gemäß Beispiel 2 | - | 2,15 g |

90 g der Soßenmischung wurden mit 1000 ml heißem Wasser aufgegossen und mit dem Schneebesen kräftig verrührt.

### Beispiel F18: Braune Soße mit erfindungsgemäßen Verkapselungsprodukten

| Bestandteil | Rezeptur A | Rezeptur B |
|---|---|---|
| Stärke | 39,00 g | 40,00 g |
| Maltodextrin | 33,00 g | 33,10 g |
| Kochsalz | 6,00 g | 4,5 g |
| Zuckerkulör, sprühgetrocknet | 5,00 g | 5,00 g |
| Hefeextraktpulver | 3,00 g | 3,00 g |
| Natriumglutamat | 2,00 g | 2,00 g |
| Zucker | 0,50 g | 0,50 g |
| Fettpulver | 5,00 g | 5,00 g |
| Tomatenpulver | 3,00 g | 3,00 g |
| Natürliches Gemüseextrakt | 1,00 g | 1,00 g |
| Zwiebelextrakt | 0,30 g | 0,30 g |
| Pfefferextrakt | 0,10 g | 0,10 g |
| Trockenaroma | 1,00 g | 1,00 g |
| Verkapselungsprodukt gemäß Beispiel 1 | 1,10 g | - |
| Verkapselungsprodukt gemäß Beispiel 2 | - | 1,50 g |

90 g der Soßenmischung wurden mit 1000 ml heißem Wasser aufgegossen und mit dem Schneebesen kräftig verrührt.

### Beispiel F19: Tomatensuppe mit erfindungsgemäßen Verkapselungsprodukten

| Bestandteil | Rezeptur A | Rezeptur B |
|---|---|---|
| Wasser | 50,20 g | 50,80 g |
| Pflanzenöl | 5,50 g | 5,50 g |
| Tomatenpaste | 24,00 g | 24,00 g |
| Sahne | 1,00 g | 2,50 g |
| Zucker | 2,00 g | 2,00 g |
| Kochsalz | 1,70 g | 1,10 g |
| Natriumglutamat | 0,40 g | 0,40 g |
| Weizenmehl | 5,50 g | 5,50 g |
| Stärke | 1,20 g | 1,20 g |
| gewürfelte Tomaten | 7,50 g | 5,50 g |
| Verkapselungsprodukt gemäß Beispiel 1 | 1,00 g | - |
| Verkapselungsprodukt gemäß Beispiel 2 | - | 1,50 g |

Die festen Bestandteile wurden eingewogen, gemischt und dem Wasser hinzugefügt. Das Pflanzenöl wurde zudosiert und die Tomatenpaste hinzugegeben. Die Mischung wurde unter Rühren aufgekocht.

### Beispiel F20: Hühnersuppe mit erfindungsgemäßen Verkapselungsprodukten

| Bestandteil | Rezeptur A | Rezeptur B |
|---|---|---|
| Wasser | Ad 100 g | Ad 100 g |
| Stärke | 1,50 g | 1,50 g |
| Hefeextrakt | 0,40 g | 0,40 g |
| Zwiebelpulver | 0,30 g | 0,30 g |
| Pfeffer | 0,03 g | 0,03 g |
| Knoblauchpulver | 0,05 g | 0,05 g |
| Gemüseextrakt | 0,20 g | 0,20 g |
| Kochsalz | 1,00 g | 0,50 g |
| Hühnersuppenaroma | 0,15 g | 0,22 g |
| Pflanzenfett | 0,50 g | 0,50 g |
| Hühnerfleisch | 7,00 g | 7,00 g |
| Suppengemüse | 14,50 g | 14,50 g |
| Nudeln | 3,00 g | 3,00 g |
| Verkapselungsprodukt gemäß Beispiel 1 | 1,00 g | - |
| Verkapselungsprodukt gemäß Beispiel 2 | - | 1,50 g |

Die festen Bestandteile wurden eingewogen, gemischt und dem Wasser hinzugefügt. Das Hühnerfett wurde zugegeben und Hühnerfleisch, Suppengemüse und Nudeln hinzugefügt. Die Mischung wurde unter Rühren aufgekocht.

### Beispiel F21: Instantsuppe mit erfindungsgemäßen Verkapselungsprodukten, Typ Lauch-Creme

| Bestandteil | A | B |
|---|---|---|
| Kartoffelstärke | 20,0 g | 21,0 g |
| Fettpulver | Ad 100 g | Ad 100 g |
| Lactose | 20,0 g | 21,0 g |
| Maltodextrin | 11,73 g | 11,70 g |
| Kochsalz | 8,0 g | 8,0 g |
| Natriumglutamat | 3,0 g | - |
| Spinatpulver | 2,0 g | 2,0 g |
| Grünes Lauchpulver | 2,0 g | 2,0 g |
| Citronensäure, als Pulver | 0,3 g | 0,3 g |
| Gehärtetes Pflanzenfett | 3,0 g | 3,0 g |
| Gefriergetrockneter Lauch | 1,0 g | 1,0 g |
| Huhnaroma | 1,0 g | 1,0 g |
| Würzmischung Typ "grüner Lauch", Pulver | 2,0 g | 2,0 g |
| Würzmischung, Typ "gekochte Zwiebel" | 0,6 g | 0,6 g |
| Hefe-Würzmischung, Typ "Gemüsebrühe", Pulver | 0,3 g | 0,8 g |
| Curcuma-Extrakt | 0,07 g | 0,07 g |
| Verkapselungsprodukt gemäß Beispiel 1 | 1,00 g | - |
| Verkapselungsprodukt gemäß Beispiel 2 | - | 1,50 g |

5 g der jeweiligen Pulvermischung wurden mit je 100 ml heißem Wasser aufgegossen, um eine verzehrfertige Suppe zu erhalten.

### Beispiel F22: Instantsuppe mit erfindungsgemäßen Verkapselungsprodukten, Typ Hühnersuppe mit Nudeln

| Bestandteil | Rezeptur A | Rezeptur B |
|---|---|---|
| Stärke | 16,0 g | 17,2 g |
| Kochsalz | 7,0 g | 7,0 g |
| Saccharose, raffiniert | 3,2 g | 3,2 g |
| Natriumglutamat | 3,2 g | - |
| Natriuminosinat / Natriumguanylat im Verhältnis 1:1 | 0,8 g | 0,8 g |
| Säurehydrolysiertes Pflanzenprotein | 8,0 g | 8,0 g |
| Fettpulver | 2,0 g | 2,0 g |
| Gemüsefett, sprühgetrocknet | 1,0 g | 1,0 g |
| Gefriergetrocknetes Hühnerfleisch, in Stückchen | 2,0 g | 2,0 g |
| Suppen-Nudeln | Ad 100 g | Ad 100 g |
| Maltodextrin | 12,16 g | 13,13 g |
| Chinesisches Gemüse, gefriergetrocknet | 4,6 g | 4,6 g |
| Huhnaroma | 8,0 g | 8,0 g |
| Lebensmittelfarbstoff Riboflavin | 0,04 g | 0,04 g |
| Verkapselungsprodukt gemäß Beispiel 1 | 1,00 g | - |
| Verkapselungsprodukt gemäß Beispiel 2 | - | 1,50 g |

5,0 g der jeweiligen Pulvermischung wurden 10 Minuten in je 100 ml Wasser gekocht, um eine verzehrfertige Suppe zu erhalten.

### Beispiel F23: Würzmischung mit erfindungsgemäßen Verkapselungsprodukten, Typ "Pfeffer":

| Bestandteil | Rezeptur A | Rezeptur B |
|---|---|---|
| Milchprotein | 0,8 g | 0,8 g |
| Johannisbrotkernmehl | 2,0 g | 2,0 g |
| Maisstärke | Ad 100 g | Ad 100 g |
| Kochsalz | 14,0 g | 15,0 g |
| Paprikapulver | 12,0 g | 13,0 g |
| Tomatenpulver | 12,0 g | 13,0 g |
| Saccharose | 4,0 g | 4,0 g |
| Knoblauchpulver | 0,5 g | 0,5 g |
| Gehärtetes Pflanzenfett | 8,0 g | 8,0 g |
| Fettpulver | 10,0 g | 11,0 g |
| Natriumglutamat | 6,0 g | 1,0 g |
| Lebensmittelfarbstoff Rote Bete und Paprika | 2,0 g | 2,0 g |
| Aroma Typ "Pfeffer" | 2,0 g | 2,0 g |
| Aroma Typ "Pizza" | 1,2 g | 1,2 g |
| Aroma Typ "Tomate" | 0,4 g | 0,4 g |
| Extrakt aus schwarzem Pfeffer | 0,1 g | 0,1 g |
| Verkapselungsprodukt gemäß Beispiel 1 | 1,00 g | - |
| Verkapselungsprodukt gemäß Beispiel 2 | - | 1,50 g |

Jeweils 100 g Nackensteak vom Schwein wurde mit jeweils 1,7 g der Zubereitungen A und B gleichmäßig bestreut und gebraten.

### Beispiel F24: Bouillon mit erfindungsgemäßen Verkapselungsprodukten

| Bestandteil | Rezeptur A | Rezeptur B |
|---|---|---|
| Fettpulver | 8,77 g | 8,77 g |
| Natriumglutamat | 8,77 g | 5 g |
| Hefeextrakt Pulver | 12,28 g | 12,28 g |
| Kochsalz | 29,83 g | 29,83 g |
| Maltodextrin | Ad 100 g | Ad 100 g |
| Natürlicher Gemüseextrakt | 3,07 g | 3,07 g |
| Verkapselungsprodukt gemäß Beispiel 1 | 1,00 g | - |
| Verkapselungsprodukt gemäß Beispiel 2 | - | 1,50 g |

15 g der jeweiligen Pulvermischung wurden mit je 1000 ml heißem Wasser aufgegossen.

### Beispiel F25: Tomatenketchup mit erfindungsgemäßen Verkapselungsprodukten

| Inhaltsstoff | A (Gew.-%) | B (Gew.-%) |
|---|---|---|
| Kochsalz | 2 | 2 |
| Stärke, Farinex WM 55 | 1 | 1 |
| Sucrose | 12 | 9,6 |
| Hesperetin 2,5%ig in 1,2-Propylenglycol | - | 0,2 |
| Tomaten-Konzentrat 2-fach | 40 | 40 |
| Glucosesirup 80 Brix | 18 | 18 |
| Branntweinessig 10% | 7 | 7 |
| Wasser | Ad 100 | Ad 100 |
| Verkapselungsprodukt gemäß Beispiel 1 | 1,00 g | - |
| Verkapselungsprodukt gemäß Beispiel 2 | - | 1,50 g |

Die Inhaltsstoffe werden in der angegebenen Reihenfolge gemischt und das fertige Ketchup mit Hilfe eines Rührwerkes homogenisiert, in Flaschen abgefüllt und sterilisiert.

### Beispiel F26: Halsbonbons mit flüssig-viskoser Kernfüllung (centre-filled hard candy)

| | I (Gew.-%) | II (Gew.-%) |
|---|---|---|
| Mischung A (Hülle) (80% der Bonbons) | | |
| Zucker (Saccharose) | 58,12 | 49,37 |
| Glucosesirup (Feststoffgehalt 80%) | 41,51 | 49,37 |
| Pfefferminzöl | 0,10 | 0,15 |
| I-Menthylsuccinat | 0,07 | 0,10 |
| I-Menthol | 0,10 | - |
| Citronenöl | 0,10 | 0,10 |
| Citronensäure | - | 0,91 |
| Total (Hülle): | 100 | 100 |

| Mischung B (Kern) (20% der Bonbons) | | |
|---|---|---|
| High Fructose Maissirup (Gehalt an festen Zuckern 85%, knapp 15% Wasser) | 83,42 | 83,38 |
| Glycerin | 15,00 | 14,50 |
| Verkapselungsprodukt gemäß Beispiel 2 | 1,00 | 1,50 |
| Lecithin | 0,02 | 0,02 |
| Zimtöl | - | 0,30 |
| Eucalyptusöl | 0,28 | - |
| Trans-Pellitorin | 0,01 | - |
| Vanillylalkohol-n-butylether | - | 0,10 |
| Roter Farbstoff, als 5%ige wässrige Lösung | 0,20 | 0,20 |
| Vanillin | 0,07 | - |
| Total (Kern): | 100 | 100 |

In Anlehnung an die in US 6,432,441 (dort Beispiel 1) sowie die in US 5,458,894 bzw. US 5,002,791 beschriebenen Verfahren wurden Bonbons mit flüssig-viskosem Kern hergestellt. Beide Mischungen A und B wurden separat zu Basen für Hülle (Mischung A) bzw. Kern (Mischung B) verarbeitet. Die mittels Co-Extrusion erhaltenen gefüllten Halsbonbons wirkten bei betroffenen Personen beim Verzehr gegen Husten, Halsschmerzen und Heiserkeit.

## Patentansprüche

1. Vacciniumfruchtextrakt-Zubereitung, umfassend:
a) einen Vacciniumfruchtextrakt,
b) einen Anteil nicht-wasserlöslicher Bestandteile eines Hefezell-Autolysats, und
c) einen Anteil wasserlöslicher Bestandteile eines Hefezell-Autolysats.

2. Zubereitung nach Anspruch 1, **dadurch gekennzeichnet, dass** ein Hefezell-Autolysat den wasserlöslichen und nicht wasserlöslichen Anteil der Zubereitung bildet.

3. Zubereitung nach Anspruch 1 oder 2, umfassend:
a) 0.5 - 50,0 Gew.-% Vacciniumfruchtextrakt,
b) 30 - 98,9 Gew.% Hefezell-Autolysat,
c) 0,1 - 10,0 Gew.% Natriumchlorid,
d) 0,1 - 10,0 Gew.-% Restwasser.

4. Zubereitung nach einem der vorherigen Ansprüche, **dadurch gekennzeichnet, dass** der Vacciniumfruchtextrakt enthält oder besteht aus: einem Extrakt von Beeren der amerikanischen Heidelbeere oder Kulturheidelbeere (Vaccinium corymbosum), Preiselbeere (Vaccinium vitis-idaea), gewöhnliche Moosbeere (Vaccinium oxycoccos), großfruchtige Moosbeere oder Kranbeere (cranberry) (Vaccinium macrocarpon), kleinfrüchtige Moosbeere (Vaccinium microcarpum), Alpen-Rauschbeere (Vaccinium gaultheroides), Rauschbeere (Vaccinium uliginosum), Big Huckleberry (Vaccinium membranaceum), Red Huckleberry (Vaccinium parvifolium), Sparkleberry (Vaccinium arboreum), Ohelo-Beere (Vaccinium reticulatum) und der kanadischen Blaubeere (Vaccinium myrtilloides), und Mischungen zweier oder mehrerer dieser Extrakte.

5. Zubereitung nach einem der vorherigen Ansprüche, **dadurch gekennzeichnet, dass** der Anteil an Anthocyanen 0,1-30 Gew.-% an der Trockensubstanz der gesamten Zubereitung beträgt.

6. Zubereitung nach einem der vorherigen Ansprüche, **dadurch gekennzeichnet, dass** das Hefezell-Autolysat ein Autolysat von Ascomyceten-Zellen
a) der Familie Saccharomycetaceae, bevorzugt deren Unterfamilie Saccharomycetoideae und besonders bevorzugt der Gattung Saccharomyces oder
b) der Familie Cryptococcaceae, bevorzugt deren Gattungen Candida, Torulopsis und/oder Kloeckera
oder eine Mischung von Autolysaten zweier oder mehrerer dieser Ascomyceten ist.

7. Zubereitung nach einem der vorherigen Ansprüche, **dadurch gekennzeichnet, dass** das Gesamt-Gewichtsverhältnis der nicht-wasserlöslichen zu den löslichen Bestandteilen des Hefezell-Autolysats 0,1 : 1 bis 9 : 1, bevorzugt 0.4 :1 bis 2,3 : 1 und besonders bevorzugt 0,7 : 1 bis 1,5 : 1 beträgt.

8. Der Ernährung oder dem Genuss dienende Zubereitung oder pharmazeutische Zubereitung, umfassend eine Zubereitung nach einem der vorherigen Ansprüche.

9. Verfahren zum Herstellen einer Zubereitung nach einem der vorherigen Ansprüche, umfassend die Schritte:
a) Bereitstellen eines Vacciniumfruchtextrakts,
b) Bereitstellen eines Anteils nicht-Wasserlöslicher Bestandteile und eines Anteil wasserlöslicher Bestandteile eines Hefezell-Autolysats, und
c) Mischen des Vacciniumfruchtextrakts mit den Hefezell-Autolysatanteilen und Wasser bei einer Temperatur zwischen 0 und 60°C für einen Zeitraum von weniger als 4 Stunden, vorzugsweise zwischen 6 und 30 min.

10. Verwendung eines Hefezell-Autolysats umfassend wasserlösliche und nicht-wasserlösliche Bestandteile als Träger eines Vacciniumfruchtextrakts.

11. Verwendung eines Hefezell-Autolysats umfassend wasserlöslichen und nicht-wasserlösliche Bestandteile zum Herstellen einer Vacciniumfruchtextrakt-Zubereitung mit einem Verhältnis der Vacciniumfruchtextrakt-Freisetzung in Magen zu Darm von 0,3: 1 bis 3 : 1.

## Claims

1. Vaccinium fruit extract preparation comprising:
a) a Vaccinium fruit extract,
b) a content of water-insoluble constituents of a yeast cell autolysate, and
c) a content of water-soluble constituents of a yeast cell autolysate.

2. Preparation according to claim 1, **characterized in that** a yeast cell autolysate forms the water-soluble and water-insoluble content of the preparation.

3. Preparation according to claim 1 or 2, comprising:
a) 0.5 - 50.0 wt.% of Vaccinium fruit extract,
b) 30 - 98.9 wt.% of yeast cell autolysate,
c) 0.1 - 10.0 wt.% of sodium chloride,
d) 0.1 - 10.0 wt.% of residual water.

4. Preparation according to one of the preceding claims, **characterized in that** the Vaccinium fruit extract comprises or consists of: an extract of berries of the North American highbush blueberry or cultivated blueberry (Vaccinium corymbosum), cowberry (Vaccinium vitis-idaea), common cranberry (Vaccinium oxycoccos), large cranberry or bearberry (Vaccinium macrocarpon), small cranberry (Vaccinium microcarpum), false berry (Vaccinium gaultheroides), bog blueberry (Vaccinium uliginosum), big huckleberry (Vaccinium membranaceum), red huckleberry (Vaccinium parvifolium), sparkleberry (Vaccinium arboreum), ohelo berry (Vaccinium reticulatum) and the Canadian blueberry (Vaccinium myrtilloides), and mixtures of two or more of these extracts.

5. Preparation according to one of the preceding claims, **characterized in that** the content of anthocyans is 0.1 - 30 wt.% of the dry matter of the total preparation.

6. Preparation according to one of the preceding claims, **characterized in that** the yeast cell autolysate is an autolysate of Ascomycetes cells
a) of the Saccharomycetaceae family, preferably the Saccharomycetoideae subfamily thereof and particularly preferably of the Saccharomyces genus or
b) of the Cryptococcaceae family, preferably the Candida, Torulopsis and/or Kloeckera genera thereof
or a mixture of autolysates of two or more of these Ascomycetes.

7. Preparation according to one of the preceding claims, **characterized in that** the total weight ratio of the water-insoluble to the soluble constituents of the yeast cell autolysate is 0.1 : 1 to 9 : 1, preferably 0.4 : 1 to 2.3 : 1 and particularly preferably 0.7 : 1 to 1.5 : 1.

8. Preparation for nutrition or consumption for pleasure or pharmaceutical preparation comprising a preparation according to one of the preceding claims.

9. Process for the production of a preparation according to one of the preceding claims, comprising the steps:
a) provision of a Vaccinium fruit extract
b) provision of a content of water-insoluble constituents and a content of water-soluble constituents of a yeast cell autolysate, and
c) mixing of the Vaccinium fruit extract with the yeast cell autolysate contents and water at a temperature of between 0 and 60 °C for a period of less than 4 hours, preferably between 6 and 30 min.

10. Use of a yeast cell autolysate comprising water-soluble and water-insoluble constituents as a carrier for a Vaccinium fruit extract.

11. Use of a yeast cell autolysate comprising water-soluble and water-insoluble constituents for production of a Vaccinium fruit extract preparation with a ratio of the release of Vaccinium fruit extract in the stomach to the intestine of from 0.3 : 1 to 3 : 1.

## Revendications

1. Préparation à base d'extrait de fruit du genre *Vaccinium,* comprenant:
a) un extrait de fruit du genre *Vaccinium,*
b) une portion de constituants non hydrosolubles d'un autolysat de cellules de levures et
c) une portion de constituants hydrosolubles d'un autolysat de cellules de levures.

2. Préparation selon la revendication 1, **caractérisée par le fait qu'**un autolysat de cellules de levures forme la portion hydrosoluble et non hydrosoluble de la préparation.

3. Préparation selon la revendication 1 ou 2, comprenant:
a) 0,5 - 50,0 % en poids d'extrait de fruit du genre *Vaccinium,*
b) 30 - 98,9 % en poids d'autolysat de cellules de levures,
c) 0,1 -10,0 % en poids de chlorure de sodium,
d) 0,1 -10,0 % en poids d'eau résiduelle.

4. Préparation selon l'une quelconque des revendications précédentes, **caractérisée par le fait que** l'extrait de fruit du genre *Vaccinium* contient ou est formé de: un extrait de baies parmi les myrtilles américaines ou myrtilles cultivées (Vaccinium corymbosum), les airelles rouges (Vaccinium vitis-idaea), les airelles canneberges (Vaccinium oxycoccos), les airelles à gros fruits ("cranberries" en anglais) (Vaccinium macrocarpon), les airelles à petits fruits (Vaccinium microcarpum), les myrtilles de loup des Alpes (Vaccinium gaultherioides), les myrtilles de loup (Vaccinium uliginosum), les airelles à feuilles membraneuses ("big huckleberries" en anglais) (Vaccinium membranaceum), les airelles à fruits rouges ("red huckleberries" en anglais) (Vaccinium parvifolium), les airelles en arbre (Vaccinium arboreum), les baies Ohelo (espèce hawaïenne) (Vaccinium reticulatum) et les airelles fausses myrtilles (Vaccinium myrtilloides), ainsi que les mélanges d'au moins deux de ces extraits.

5. Préparation selon l'une quelconque des revendications précédentes, **caractérisée par le fait que** la portion d'anthocyanes représente entre 0,1 et 30 % en poids de la substance sèche de la préparation totale.

6. Préparation selon l'une quelconque des revendications précédentes, **caractérisée par le fait que** l'autolysat de cellules de levures est un autolysat de cellules de levures ascomycètes
a) de la famille des Saccharomycetaceae, de préférence de la sous-famille des Saccharomycetoideae et, encore plus préférablement, du genre Saccharomyces ou
b) de la famille des Cryptococcaceae, de préférence des genres Candida, Torulopsis et/ou Kloeckera
ou un mélange d'autolysats d'au moins deux de ces ascomycètes.

7. Préparation selon l'une quelconque des revendications précédentes, **caractérisée par le fait que** le rapport pondéral global entre les constituants non hydrosolubles et les constituants solubles de l'autolysat de cellules de levures est compris entre 0,1:1 et 9:1, de préférence entre 0,4:1 et 2,3:1 et, encore plus préférablement, entre 0,7:1 et 1,5:1.

8. Préparation à usage nutritif ou gustatif ou préparation pharmaceutique, comprenant une préparation selon l'une quelconque des revendications précédentes.

9. Procédé de fabrication d'une préparation selon l'une quelconque des revendications précédentes, comprenant les étapes:
a) mise à disposition d'un extrait de fruit du genre Vaccinium,
b) mise à disposition d'une portion de constituants non hydrosolubles et d'une portion de constituants hydrosolubles d'un autolysat de cellules de levures et
c) mélange de l'extrait de fruit du genre Vaccinium avec les portions d'autolysat de cellules de levures et de l'eau à une température comprise entre 0 et 60°C pour une période de temps inférieure à 4 heures, de préférence entre 6 et 30 minutes.

10. Utilisation d'un autolysat de cellules de levures comprenant des constituants hydrosolubles et non hydrosolubles comme supports d'un extrait de fruit du genre Vaccinium.

11. Utilisation d'un autolysat de cellules de levures comprenant des constituants hydrosolubles et non hydrosolubles pour la fabrication d'une préparation à base d'extrait de fruit du genre Vaccinium, dont le rapport de libération de l'extrait de fruit du genre Vaccinium dans l'estomac par rapport à l'intestin est compris entre 0,3:1 et 3:1.
